(19) Europäisches Patentamt
European Patent Office
Office européen des brevets

(11) Publication number : **0 345 846 B1**

# (12) EUROPEAN PATENT SPECIFICATION

(45) Date of publication of patent specification : **26.08.92 Bulletin 92/35**

(51) Int. Cl.$^5$ : **C07C 309/29**

(21) Application number : **89201285.7**

(22) Date of filing : **19.05.89**

## (54) Process for the preparation of surfactants.

(30) Priority : **07.06.88 GB 8813427**

(43) Date of publication of application : **13.12.89 Bulletin 89/50**

(45) Publication of the grant of the patent : **26.08.92 Bulletin 92/35**

(84) Designated Contracting States : **BE DE FR GB NL**

(56) References cited : **EP-A- 0 060 079**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Stapersma, Johan**
**Badhuisweg 3**
**NL-1031 CM Amsterdam (NL)**
Inventor : **Van Ginkel, Roelof**
**Badhuisweg 3**
**NL-1031 CM Amsterdam (NL)**
Inventor : **Verkouw, Hendrik Tijmen**
**Badhuisweg 3**
**NL-1031 CM Amsterdam (NL)**
Inventor : **Huiden, Maria Antonia**
**Badhuisweg 3**
**NL-1031 CM Amsterdam (NL)**

**EP 0 345 846 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to an improved process for the preparation of anionic surfactants containing alkyl, alkoxy, aryl and sulfonate groups, wherein the sulfonate group-bearing aryl group is connected to the alkyl group by means of an alkoxy group.

Surfactants as hereinbefore described are known from EP 0 060 079, and are potentially useful in the recovery of oil from subterranean reservoirs. However, the methods following which these surfactants may be prepared and which have also been described in EP 0 060 079, include a number of aspects which, as will be outlined hereinafter, will almost certainly be a restriction for a technical scale preparation of said surfactants. This is considered to be a disadvantage for products which have been developed for large scale use, i.e. for use in the enhanced oil recovery.

A number of scale-limiting aspects observed with the known methods of preparation are:

a) the use of non-readily available starting compounds and/or the use of rather expensive reagents to prepare said starting compounds,

b) the use of a phase separation step wherein the organic phase includes a considerable amount of $CHCl_3$, introduces a potential health hazard,

c) purification of the organic phase as mentioned under b) with NaOH (3x) and water (3x) followed by drying with $MgSO_4$,

d) removing the $CHCl_3$ via evaporation, and

e) employing chromatographic techniques to isolate the desired product.

Hence it can be concluded that there is need for improvement in the preparation of the surfactants as described hereinbefore.

The problem underlying the present invention is finding a process for the preparation of said surfactants which does not include one or more of the scale-limiting aspects mentioned hereinbefore and thus allows preparation on a technical scale.

As a result of continuing and extensive research and experimentation there was surprisingly found that it is possible to prepare surfactants of the hereinbefore mentioned type, on a technical scale, by selecting a sulfate-type compound as one of the starting compounds.

The invention provides therefore a process for the preparation of compounds of general formula

$$[R(OCH_2\underset{\displaystyle CH_3}{\underset{|}{C}H})_m(OCH_2CH_2)_nOC_6H_2(R^1)_2SO_3]_pM \quad (I)$$

which comprises

a) the reaction of a sulfate compound of general formula

$$[R(OCH_2\underset{\displaystyle CH_3}{\underset{|}{C}H})_m(OCH_2CH_2)_nOSO_3]_pM \quad (II)$$

with an aromatic alcohol of general formula $C_6H_3(R^1)_2OH$ (III) or an aromatic sulfonate of general formula$[C_6H_2(R^1)_2(OH)SO_3]_p$ M (IV), in which formulae R is a linear or branched alkyl group having on average from 8-22 carbon atoms; each $R^1$ individually is H, a lower alkyl group, an alkoxy group, a thioalkoxy group, or halogen, or when both $R^1$'s are ortho-positioned with respect to one another, both $R^1$'s together may form a group

$$-\underset{R^1}{\underset{|}{C}}=\underset{R^1}{\underset{|}{C}}-\underset{R^1}{\underset{|}{C}}=\underset{R^1}{\underset{|}{C}}-$$

wherein each $R^1$ individually has the same meaning as hereinbefore; M is an alkali metal, ammonium or an alkaline earth metal; p is 1 or 2;

$m+n \leqq 15$, wherein $m \geqq 0$ and $n>0$;

in the presence of a base,

b) sulfonating and neutralizing the reaction product as prepared under a) for the preparation of which a compound of general formula III has been used.

In the process of the present invention wherein in step a) a compound of general formula II (hereinafter referred to as compound II) is reacted with a compound of general formula III or IV respectively (hereinafter referred to as compounds III or IV), the molar ratios wherein said compounds may be used are not critical and may vary over wide ranges. It is preferred however, for the compounds III or IV respectively to be present in an at least equimolar amount with respect to that of compound II. Especially preferred is a process wherein the molar ratio of compound II to compound III or IV respectively is in the range of from 0.55:1 to 0.95:1. In practice the compounds III will generally not have more than 3 aromatic nuclei per molecule.

The base, in the presence of which the reaction between compound II and III or IV respectively, is conducted is generally used in such an amount that the ratio of moles of compound II to equivalents of said base is not more than 0.99:1, preferably the ratio of moles of compound II to equivalents of base is in the range of from 0.55:1 to 0.95:1.

Preferably the base is an inorganic base such as an alkali metal, an alkaline earth metal or ammonium hydroxide.

The reaction between compounds II and III or IV respectively is preferably conducted in an aqueous medium in an autoclave at a temperature in the range of from 150-200 °C and more preferably at a temperature in the range of from 165-190 °C. The aqueous medium may be provided by the addition of water to the reactor and/or by introducing the compound II as an aqueous solution or dispersion, which solution or dispersion will generally have a compound II content in the range of from 10-85 %m. The base may be introduced into the reactor as such, as an aqueous solution or as the reaction product with the compound III or IV respectively.

When the amount of water in the reactor is relatively large vis-à-vis the amount of compound II, which may be the case e.g. when compound II has been introduced as an aqueous solution or dispersion having a relatively low compound II content, it has been found to be beneficial to employ a large excess of base and of compound III or IV respectively with respect to compound II, and vice versa.

At the end of the reaction as described hereinbefore, the reactor contents are cooled without stirring to a temperature in the order of 50 °C. When the reactor contains a substantial amount of water-insoluble matter, the cooled reactor contents will be present as a two-phase system, i.e. an organic upper phase containing the water-insoluble or virtually water-insoluble matter and an aqueous lower phase containing water-soluble matter.

The formation of water-insoluble or virtually water-insoluble product is determined by the fact whether a compound III or a compound IV is used in step a) of the present process - the use of compound IV resulting in the formation of a water-soluble reaction product - as well as by the nature of compound II. With the compounds II it is desirable that there is a certain balance between the number of carbon atoms in the alkyl chain R and value of m+n, in that the number of carbon atoms is more or less proportional to the value of m+n; i.e. when said number of carbon atoms is small or large the value of m+n should be low or high respectively. When in the process of the present invention a compound II is used having said balance between the number of carbon atoms in R and the value of m+n, or when using a compound II wherein m+n is small, the reaction mixture containing the reaction product thereof with a compound III (hereinafter referred to as alkyl alkoxy aryl ether) will result in a two-phase system. The specific composition of compounds I and II with respect to the number of carbon atoms in R and the value of m+n will generally be governed by the end-use of compound I.

When a compound II is reacted with a compound IV it has been experienced that in the course of said reaction quite a considerable part of compound II may be hydrolysed to the corresponding alkylalkoxylate. The ultimate reaction product may thus contain, in addition to the desired compound I, alkylalkoxylate, unreacted compound II and IV and sulfate. It is realised that such a mixture, from which the desired compound I is not easily isolated, will not always be acceptable for all purposes. Hence it is preferred in step a) of the process of the present invention to react a compound II with a compound III. In this preferred embodiment of the process it is preferred to employ such compounds II which will result in the formation of water-insoluble alkyl alkoxy aryl ethers and thus upon cooling provide a two-phase system.

The organic phase which remains after separating-off the aqueous phase, may contain some excess compound III as the corresponding alcoholate. When the presence of said aromatic alcoholate or the amount wherein it is present, is less desirable or even unacceptable, the concentration of said compound can be reduced to almost any desired value at reduced pressure and at elevated temperature by known methods, after first having been acidified in order to convert the aromatic alcoholate compound to the corresponding compound III. After removal of the compound III the organic phase will generally contain from 90-97 %m of the alkyl alkoxy aryl ether, and from 10-3 %m of alkyl alkoxylate resulting from hydrolysis of said compound II, hereinafter referred to as parent alkyl alkoxylate.

The preparation of the desired compound I according to the preferred embodiment of the present invention, i.e. via the formation of the intermediate alkyl alkoxy aryl ether, requires a consecutive step wherein said alkyl

alkoxy aryl ether is modified to contain a sulfonate group. In principle the method employed for the introduction of said sulfonate group is not critical and may include sulfonation by means of gaseous or liquid sulfur trioxide ($SO_3$), oleum, and chlorosulfonic acid. Preferably the sulfonation is conducted with gaseous $SO_3$ and it is especially preferred to conduct the sulfonation of the alkyl alkoxy aryl ethers, as hereinbefore described, with gaseous $SO_3$ in a falling film reactor, a reactor known e.g. from U.S. Patent 4.059.620. With said method the organic substrate to be sulfonated, is introduced continuously as a liquid into a vertically positioned $SO_3$-resistant tube, equipped with an external cooling jacket, and allowed to flow down along the inside surface of said tube as a thin film. Cocurrently a gaseous mixture of $SO_3$ and an inert gas, for which nitrogen or dry air may conveniently be employed, is also allowed to flow down through said tube. Conveniently the $SO_3$ content of the gas mixture will be in the range of from 0.5-8 %v and preferably in the range of from 2.5-4,0 %v. The ratio wherein $SO_3$ and the organic substrate to be sulfonated will be employed, is dependent on the nature of said substrate. Generally the molar ratio of $SO_3$ to organic substrate, i.e. alkyl alkoxy aryl ether, will be in the range of from 1.05:1 to 1.6:1. The $SO_3$/organic substrate ratio required for a given substrate can conveniently be determined by analyzing the effluent from the falling film reactor via a titration method as described hereinafter, followed by adjusting said ratio if required.

The temperature at which the sulfonation may be conducted is not critical and may vary over wide ranges and will be largely determined by the temperature of the cooling water in the reactor. Hence in the context of the present invention the sulfonation temperature will be expressed by the temperature of the cooling water in the falling film reactor. Conveniently the sulfonation with gaseous $SO_3$ is conducted in a falling film reactor wherein the cooling water has a temperature in the range of from 20-120 °C, although higher and lower temperatures are not excluded, preferably said temperature is in the range of from 50-80 °C.

If required, the effluent from the falling film reactor may first be collected in a postreactor to extend the reaction time between $SO_3$ and the alkyl alkoxy aryl ether, thereby possibly increasing the degree of sulfonation, before being fed into a gas/liquid separator.

The desired compounds I may be obtained by neutralizing the liquid bottom fraction from the gas/liquid separator to a pH e.g. in the range of from 8-9, for which a metal M based base, as described hereinbefore may be used. As the alkyl alkoxylate, which is generally present next to the alkyl alkoxy aryl ether to be sulfonated, will also react with $SO_3$ to form the corresponding alkyl alkoxy sulfate, the compounds I are generally obtained as mixtures with the corresponding alkyl alkoxy sulfate, wherein the weight ratio of compound I to alkyl alkoxy sulfate is generally in the range of from 90:10 to 97:3.

The compounds used as starting compounds in the process of the present invention i.e. compounds II, III and/or IV are compounds which are commercially available or else can be prepared via known and simple processes. A number of the alkyl alkoxy sulfates (compounds II) are commercially available e.g. from Shell Chemicals Ltd. or else can be made from their commercially available precursors following methods as described e.g. in Technical Bulletin DI 3.2.10 ex Shell International Chemical Company. The aromatic alcohols and aromatic sulfonate type compounds are commercially available from various sources.

In the process of the present invention it is preferred to employ compounds wherein m+n has on average a value in the range of from 1-7 and to use compounds III and/or IV wherein at least one $R^1$ represents H.

The invention will be further illustrated by the following examples for which the following information is provided:

## Product analysis

1) The composition of the alkyl alkoxy aryl ether reaction product was determined by means of $^1$H-NMR spectroscopy and GLC.

2) The aromatic alcohol content of the reaction product of step a) was determined via Gas Liquid Chromatography after acidification with concentrated sulfuric acid.

3) The composition of the effluent from the falling film reactor, was analyzed via the nonaqueous (ethanolic) titration method for sulfuric acid and alkylbenzene sulfonic acids in detergent intermediates, as described by Shinichiro Yamaguchi, et al. in Journal of the American Oil Chemists' Society, Vol. 55, March 1978, pp 359-362. This method is also employed for determining the correct $SO_3$/organic substrate ratio for the sulfonation of the alkyl alkoxy aryl ethers.

4) The reaction product of a compound II and a compound IV was analyzed by titration of the active matter according to ASTM D 1681-23, using hyamine.

## Alkyl alkoxy sulfate (compounds II)

The majority of the experiments were conducted with commercially available grades of alkyl ethoxy sulfates, i.e. Dobanol ethoxy sulfates, ex Shell Chemicals Ltd., the composition of said products being given by a formula such as Dobanol 25-3S/70, wherein Dobanol 25 refers to an alifatic $C_{12-15}$ alcohol whereon these products are based, 3 refers to the average number of ethoxy units per molecule, S indicates that it is a sodium sulfate and 70 the percentage by weight in water.

When commercial grades were not available, the required sulfate compounds were prepared from their commercially available precursors by reactions as have been described e.g. in Shell Chemicals Ltd. brochure: "Dobanol Information".

Example I

Preparation of $C_{12-15}(OCH_2CH_2)_3OC_6H_5$

An alkyl ethoxy aryl ether as hereinbefore described was prepared in a 5 l Renato Brignole AU 5 stainless steel autoclave by dissolving 160 g NaOH in 640 g water in said autoclave which was followed by the addition of 380 g phenol and 2016 g of a 70 %m solution in water of a $C_{12-15}(OCH_2CH_2)_3OSO_3Na$ (Dobanol 25-3S/70 ex Shell Chemicals Ltd.). Subsequently the reactor was closed and with stirring the reactor contents were heated to 180 °C and the reaction was allowed to proceed for 18 hrs at this temperature. At the end of the reaction period the reactor contents were cooled to 50 °C without stirring and were allowed to settle. This was followed by separating off the lower, aqueous phase. Analysis of the organic phase indicated a yield of compound II derivatives, i.e. alkyl alkoxy aryl ether and the parent alkyl ethoxylate of 97 %, which compounds were present in a weight ratio of 93:7; the phenol content was found to be 2.5 %m.

Examples II-XXI

Preparation of alkyl ethoxy aryl ethers of formula $R(OCH_2CH_2)_nOC_6H_5$

Following the procedure as described in Example I similar alkyl ethoxy aryl ethers ($R(OCH_2CH_2)_n OC_6H_5$) were prepared but employing alkyl ethoxy sulfates ($R(OCH_2CH_2)_n OSO_3Na$), having different values for R and/or n, and phenol or substituted phenol.

The values for R, n of the alkyl ethoxy sulfates and the type of phenol used in the preparation of the aryl ethers, are given in Table 1.

The final reaction products were found to comprise, for at least 94 %m, mixtures of the desired alkyl ethoxy aryl ether and the parent alkyl ethoxylate, which compounds were present in a weight ratio in the range of from 90:10 to 96:4; the content of phenolic type compound varied from 1.5-3.0 %m.

Table 1

| Example | R | n* | Ar |
|---|---|---|---|
| II | 12-15 | 3 | phenol |
| III | 12-15 | 3 | p-cresol |
| IV | 12-15 | 3 | o-methoxyphenol |
| V | 12-15 | 3 | o-cresol |
| VI | 12-15 | 3 | o-chlorophenol |
| VII | 12-15 | 5 | phenol |
| VIII | 12-15 | 1 | phenol |
| IX | 12-15 | 4 | phenol |
| X | 12-15 | 5 | p-cresol |
| XI | 12-15 | 3 | phenol |
| XII | 12-15 | 3 | p-t-butylphenol |
| XIII | 12-15 | 5 | p-t-butylphenol |
| XIV | 16 | 3 | phenol |
| XV | 16 | 5 | phenol |
| XVI | 18 | 3 | phenol |
| XVII | 18 | 5 | phenol |
| XVIII | 16-18 | 5 | phenol |
| XIX | 20 | 3 | phenol |
| XX | 20 | 5 | phenol |
| XXI | 22 | 7 | phenol |

* average value

Examples XXII-XXV

Preparation of sulfonated alkyl ethoxy aryl ethers in a laboratory scale falling film reactor

The alkyl ethoxy aryl ethers as prepared in Examples I, III, VII and IX were acidified with 96 %m sulfuric acid to arrive at a pH in the range of from 4-6 in order to convert any aromatic alcoholate to the corresponding aromatic alcohol, and subsequently the aromatic alcohol was stripped off in a rotary film evaporator to an aromatic alcohol content <1 %m.

The stripped alkyl ethoxy aryl ethers were subsequently fed into a laboratory scale falling film reactor (a glass reactor having an internal diameter of 5 mm and a length of 1 m) at a rate of 1 mole/h and allowed to flow down through the reactor. Cocurrently a gaseous $SO_3$/dry nitrogen mixture containing 3 %v $SO_3$ was fed into the reactor at such a rate that the $SO_3$/organic substrate molar ratio corresponded with the value given in Table 2 hereinafter. The falling film reactor was operated with cooling water having a temperature of 70 °C.

The effluent from the falling film reactor was collected in a postreactor and remained therein for on average approximately 7 min. Subsequently the post-reacted product was fed into a gas/liquid separator to remove excess $SO_3$ which was followed by analyzing the degree of sulfonation. Finally the sulfonated products were neutralized with 10 %m NaOH to a pH 8-9. The resulting data are given in Table 2.

Table 2

| Example | Aryl ether from Example | $SO_3$/substrate mol.ratio | Temp., °C | Degree of sulfonation of aryl ether, % |
|---|---|---|---|---|
| XXII | I | 1.3 | 70 | 100 |
| XXIII | III | 1.5 | 70 | 95 |
| XXIV | VII | 1.4 | 70 | 100 |
| XXV | IX | 1.4 | 70 | 100 |

Example XXVI

Large scale preparation of $C_{12-15}(OCH_2CH_2)_3OC_6H_5SO_3Na$

5.05 kg NaOH was dissolved in 20.21 kg water in a 150 l UHDE reactor, subsequently 12 kg phenol and 63.66 kg of Dobanol 25-3S/70 were added to the reactor. The reactor was closed and under stirring the reactor contents were heated to 180 °C, which resulted in a pressure within the reactor of 10 bar. After 24 hours at 180 °C the reactor contents were cooled to 100 °C whereupon the stirrer was stopped and phase separation was allowed to occur. Before separating off the aqueous layer (59.8 kg), the reactor contents were further cooled to 50 °C. After acidification with 95 g 96 %m $H_2SO_4$ to a pH=4, the organic phase was found to have a phenol content of 2.5 %m (which was determined via gas liquid chromatography). The acidified organic phase was subsequently topped to remove phenol, which topping operation was conducted in two steps in a Leybold wiped film evaporator (evaporating surface 0.3 $m^2$ ). The first step was conducted at 130 °C and 3 mbar reducing the phenol content to 1 %m, while the second evaporation step was conducted at 140 °C and 1 mbar. No phenol could be found in the 36.5 kg bottom fraction resulting from the second topping operation.
GLC analysis indicated the reaction product to be a mixture of $C_{12-15}(OCH_2CH_2)_3OC_6H_5$ and the parent ethoxylate in a molar ratio of 93:7.

Subsequently this reaction product was heated to 59 °C and fed into a falling film reactor, having a length of 2 m and an internal diameter of 9 mm, at a ratio of 4000 g/h. Cocurrently a stream of gaseous $SO_3$ (293 l/h) and dry air (9000 l/h) was fed into the reactor, the gaseous mixture having a temperature of 43 °C, providing a $SO_3$/organic substrate molar ratio of 1.27. The effluent from the falling film reactor was fed into a gas/liquid separator. Upon analysis of the liquid bottom fraction of the separator, the degree of sulfonation of the alkyl ethoxy aryl ether was found to be 100 %. Finally said bottom product was neutralized to a pH 8-9 using an aqueous 15 %m NaOH solution.

Example XXVI

Preparation of $C_{12-15}(OCH_2CH_2)_3OC_6H_5SO_3Na$ via the reaction of $C_{12-15}(OCH_2CH_2)_3OSO_3Na$ and $HOC_6H_5SO_3Na$.

In a stirred 1 l autoclave 20 g NaOH was dissolved in 300 g water, which was followed by addition of 252 g Dobanol 25-3S/70 and 98 g sodium p-phenol sulfonate. The reactor was closed and the contents heated to 180 °C and stirred at said temperature for 18 hours after which the reactor contents were cooled to 20 °C. The reaction product was found to contain $C_{12-15}(OCH_2CH_2)_3OC_6H_5SO_3Na$ and the parent ethoxylate in a 50:50 molar ratio.

## Claims

1. A process for the preparation of compounds of general formula

$$[R(OCH_2\underset{\underset{\displaystyle CH_3}{|}}{C}H)_m(OCH_2CH_2)_nOC_6H_2(R^1)_2SO_3]_pM \quad (I)$$

which comprises:

a) the reaction of a sulfate compound of general formula

$$[R(OCH_2\underset{\underset{\displaystyle CH_3}{|}}{C}H)_m(OCH_2CH_2)_nOSO_3]_pM \quad (II)$$

with an aromatic alcohol of general formula $C_6H_3(R^1)_2OH$ (III) or an aromatic sulfonate of general formula $[C_6H_2(R^1)_2(OH)SO_3]_p$ M (IV), in which formulae R is a linear or branched alkyl group having on average from 8-22 carbon atoms; each $R^1$ individually is H, a lower alkyl group, an alkoxy group, a thioalkoxy group or halogen, or when both $R^1$'s are ortho-positioned with respect to one another, both $R^1$'s together may form a group

$$-\underset{\underset{\displaystyle R^1}{|}}{C}=\underset{\underset{\displaystyle R^1}{|}}{C}-\underset{\underset{\displaystyle R^1}{|}}{C}=\underset{\underset{\displaystyle R^1}{|}}{C}-$$

wherein each $R^1$ individually has the same meaning as hereinbefore;
M is an alkali metal, ammonium or an alkaline earth metal;
p is 1 or 2; $m+n \leqq 15$, wherein $m \geqq 0$ and $n > 0$;
in the presence of a base,

b) sulfonating and neutralizing the reaction product as prepared under a) for the preparation of which a compound of general formula III has been used.

2. A process as claimed in claim 1, wherein the compounds III or IV respectively are used in an at least equimolar amount with respect to that of compound II.

3. A process as claimed in claim 2, wherein the molar ratio of compound II to compounds III or IV respectively is in the range of from 0.55:1 to 0.95:1.

4. A process as claimed in any one of claims 1-3, wherein the base is used in such an amount that the ratio of moles of compound II to equivalents of said base is not more than 0.99:1.

5. A process as claimed in claim 4, wherein the ratio is in the range of from 0.55:1 to 0.95:1.

6. A process as claimed in any one of claims 1-5, wherein the base is an inorganic base.

7. A process as claimed in claim 6, wherein the inorganic base is an alkalimetal hydroxide.

8. A process as claimed in any one of claims 1-7, wherein step a) is conducted in an aqueous medium.

9. A process as claimed in any one of claims 1-8, wherein the reaction between a compound II and a compound III or IV respectively is conducted at a temperature in the range of from 150-200 °C.

10. A process as claimed in claim 9, wherein the temperature is in the range of from 165-190 °C.

11. A process as claimed in any one of claims 1-10, wherein a compound III is employed.

**12.** A process as claimed in claim 11, wherein a compound II is used which after reaction with compound III will result in a two-phase liquid reaction mixture.

**13.** A process as claimed in claim 11 or 12, wherein gaseous sulfur trioxide is used for the sulfonation of the alkyl alkoxy aryl ether reaction product of step a).

**14.** A process as claimed in claim 13, wherein the sulfonation is conducted in a falling film reactor.

**15.** A process as claimed in claim 14, wherein the sulfur trioxide comprises from 2,5-4.0 %v of the gaseous mixture.

**16.** A process as claimed in any one of claims 13-15, wherein the molar ratio of sulfur trioxide to alkyl alkoxy aryl ether is in the range of from 1.05:1 to 1.6:1.

**17.** A process as claimed in any one of claims 13-16, wherein the sulfonation is conducted in a falling film reactor wherein the cooling water temperature is in the range of from 20-120 °C.

**18.** A process as claimed in claim 17, wherein the temperature is in the range of from 50-80 °C.

**19.** A process as claimed in any one of claims 1-18, wherein in the general formulae I, III and IV at least one $R^1$ represents H.

**20.** A process as claimed in any one of claims 1-19, wherein in general formula II the average value of m+n is in the range of from 1-7.

## Patentansprüche

**1.** Verfahren zur Herstellung von Verbindungen der allgemeinen Formel

$$[R(OCH_2\underset{\displaystyle CH_3}{CH})_m(OCH_2CH_2)_nOC_6H_2(R^1)_2SO_3]_pM \qquad (I)$$

umfassend:

a) die Umsetzung einer Sulfatverbindung der allgemeinen Formel

$$[R(OCH_2\underset{\displaystyle CH_3}{CH})_m(OCH_2CH_2)_nOSO_3]_pM \qquad (II)$$

mit einem aromatischen Alkohol der allgemeinen Formel

$$C_6H_3(R^1)_2OH \qquad (III)$$

oder einem aromatischen Sulfonat der allgemeinen Formel

$$[C_6H_2(R^1)_2(OH)SO_3]_pM \qquad (IV)$$

wobei in den Formeln R eine linear oder verzweigte Alkylgruppe mit im Mittel 8 bis 22 Kohlenstoffatomen bedeutet, jedes $R^1$ unabhängig H, eine niedere Alkylgruppe, eine Alkoxygruppe, eine Thioalkoxygruppe oder Halogen bedeutet, oder, wenn beide Reste $R^1$ sich in o-Stellung zueinander befinden, beide Reste

$R^1$ zusammen eine Gruppe

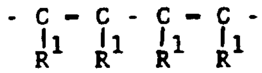

bilden können, in der jedes $R^1$ unabhängig die gleiche Bedeutung hat wie oben angegeben, M ein Alkalimetall, Ammonium oder ein Erdalkalimetall ist, p 1 oder 2 bedeutet, $m+n \leqq 15$ ist, wobei $m \geqq 0$ und $n > 0$ ist,
in Gegenwart einer Base,
b) Sulfonieren und neutralisieren des unter a) erhaltenen Reaktionsproduktes, zu dessen Herstellung eine Verbindung der allgemeinen Formel III angewandt worden ist.

**2.** Verfahren nach Anspruch 1, wobei die Verbindungen III bzw. IV in mindestens äquimolarer Menge, bezogen auf die Verbindung II, angewandt werden.

**3.** Verfahren nach Anspruch 2, wobei das Molverhältnis von Verbindung II zu Verbindungen III bzw. IV im Bereich von 0,55:1 bis 0,95:1 liegt.

**4.** Verfahren nach einem der Ansprüche 1 bis 3, wobei die Base in einer solchen Menge verwendet wird, daß das Verhältnis Mole der Verbindung II zu Äquivalente der erwähnten Base nicht mehr als 0,99:1 ist.

**5.** Verfahren nach Anspruch 4, wobei das Verhältnis im Bereich von 0,55:1 bis 0,95:1 liegt.

**6.** Verfahren nach einem der Ansprüche 11 bis 5, wobei die Base eine anorganische Base ist.

**7.** Verfahren nach Anspruch 6, wobei die anorganische Base ein Alkalimetallhydroxid ist.

**8.** Verfahren nach einem der Ansprüche 1 bis 7, wobei Stufe a) in einem wäßrigen Medium durchgeführt wird.

**9.** Verfahren nach einem der Ansprüche 1 bis 8, wobei die Reaktion zwischen einer Verbindung II und einer Verbindung III bzw. IV bei einer Temperatur im Bereich von 150 bis 200°C durchgeführt wird.

**10.** Verfahren nach Anspruch 9, wobei die Temperatur im Bereich von 165 bis 190°C liegt.

**11.** Verfahren nach einem der Ansprüche 1 bis 10, wobei eine Verbindung III angewandt wird.

**12.** Verfahren nach Anspruch 11, wobei eine Verbindung II angewandt wird, die nach Umsetzung mit der Verbindung III zu einem zweiphasigen flüssigen Reaktionsgemisch führt.

**13.** Verfahrenf nach Anspruch 11 oder 12, wobei gasförmiges Schwefeltrioxid für die Sulfonierung des AlkylalkoxyaryletherReaktionsproduktes der Stufe a) angewandt wird.

**14.** Verfahren nach Anspruch 13, wobei die Sulfonierung in einem Rieselfilmreaktor durchgeführt wird.

**15.** Verfahren nach Anspruch 14, wobei das Schwefeltrioxid 2,5 bis 4,0 Vol.-% des gasförmigen Gemisches ausmacht.

**16.** Verfahren nach einem der Ansprüche 13 bis 15, wobei das Molverhältnis von Schwefeltrioxid zu Alkylalkoxyarylether im Bereich von 1,05:1 bis 1,6:1 liegt.

**17.** Verfahren nach einem der Ansprüche 13 bis 16, wobei die Sulfonierung in einem Rieselfilmreaktor durchgeführt wird, bei dem die Kühlwassertemperätur im Bereich von 20 bis 120°C liegt.

**18.** Verfahren nach Anspruch 17, wobei die Temperatur im Bereich von 50 bis 80°C liegt.

**19.** Verfahren nach einem der Ansprüche 1 bis 18, wobei bei den allgemeinen Formeln I, III und IV mindestens ein $R^1$ H bedeutet.

20. Verfahren nach einem der Ansprüche 1 bis 19, wobei bei der allgemeinen Formel II der Mittelwert von m+n im Bereich von 1 bis 7 liegt.

## Revendications

1. Procédé de préparation de composés de formule générale :

$$[R(OCH_2\underset{CH_3}{\underset{|}{C}}H)_m(OCH_2CH_2)_nOC_6H_2(R^1)_2SO_3]_pM \qquad (I)$$

selon lequel :

a) on fait réagir un sulfate de formule générale :

$$[R(OCH_2\underset{CH_3}{\underset{|}{C}}H)_m(OCH_2CH_2)_nOSO_3]_pM \qquad (II)$$

avec un alcool aromatique de formule générale :

$$C_6H_3(R^1)_2OH \qquad (III)$$

ou un sulfonate aromatique de formule générale :

$$[C_6H_2(R^1)_2(OH)SO_3]_pM \qquad (IV)$$

dans lesquelles R représente un groupe alkyle linéaire ou ramifié comportant en moyenne de 8 à 22 atomes de carbone ; chaque groupe $R^1$ représente individuellement un atome d'hydrogène, un groupe alkyle inférieur, un groupe alkoxy, un groupe thioalkoxy ou un atome d'halogène, ou lorsque les deux groupes $R^1$ sont en position ortho l'un par rapport à l'autre, les deux groupes $R^1$ peuvent former ensemble un groupe

$$-\underset{R^1}{\underset{|}{C}}=\underset{R^1}{\underset{|}{C}}-\underset{R^1}{\underset{|}{C}}=\underset{R^1}{\underset{|}{C}}-$$

dans lequel chaque groupe $R^1$ a individuellement la même définition que ci-dessus ;
M représente un métal alcalin, un ammonium ou un métal alcalino-terreux ; p est égal à 1 ou 2 ;
$m+n \leqq 15$, avec $m \geqq 0$ et $n > 0$ ; en présence d'une base,

b) on sulfone et on neutralise le produit de réaction préparé en a) pour la préparation duquel on a employé un composé de formule générale III.

2. Procédé selon la revendication 1, dans lequel les composés respectivement III ou IV, sont employés selon une quantité au moins équimolaire par rapport à celle du composé II.

3. Procédé selon la revendication 2, dans lequel le rapport molaire du composé II aux composés respectivement III ou IV, est de 0,55:1 à 0,95:1.

4. Procédé selon l'une quelconque des revendications 1 à 3, dans lequel la base est employée selon une quantité telle que le rapport du nombre de moles du composé II au nombre d'équivalents de la base, ne soit pas supérieur à 0,99:1.

5. Procédé selon la revendication 4, dans lequel le rapport est de 0,55:1 à 0,95:1.

6. Procédé selon l'une quelconque des revendications 1 à 5, dans lequel la base est une base inorganique.

7. Procédé selon la revendication 6, dans lequel la base inorganique est un hydroxyde de métal alcalin.

8. Procédé selon l'une quelconque des revendications 1 à 7, dans lequel l'étape a) est effectuée dans un milieu aqueux.

9. Procédé selon l'une quelconque des revendications 1 à 8, dans lequel la réaction entre un composé II et un composé respectivement III ou IV, est effectuée à une température de 150 à 200 °C.

10. Procédé selon la revendication 9, dans lequel la température est de 165 à 190 °C.

11. Procédé selon l'une quelconque des revendications 1 à 10, dans lequel on emploie un composé III.

12. Procédé selon la revendication 11, dans lequel on emploie un composé II qui, après réaction avec un composé III, résulte en un mélange réactionnel liquide biphasique.

13. Procédé selon la revendication 11 ou 12, dans lequel on emploie du trioxyde de soufre gazeux pour la sulfonation du produit de réaction à base d'alkyl alkoxy aryl éther de l'étape a ) .

14. Procédé selon la revendication 13, dans lequel la sulfonation est effectuée dans un réacteur à film tombant.

15. Procédé selon la revendication 14, dans lequel le trioxyde de soufre représente de 2,5 à 4,0 % en volume du mélange gazeux.

16. Procédé selon l'une quelconque des revendications 13 à 15, dans lequel le rapport molaire du trioxyde du soufre à l'alkyl alkoxy aryl éther, est de 1,05:1 à 1,6:1.

17. Procédé selon l'une quelconque des revendications 13 à 16, dans lequel la sulfonation est effectuée dans un réacteur à film tombant, dans lequel la température de l'eau de refroidissement est de 20 à 120 °C.

18. Procédé selon la revendication 17, dans lequel la température est de 50 à 80 °C.

19. Procédé selon l'une quelconque des revendications 1 à 18, dans lequel, dans les formules générales I, III et IV, au moins un groupe $R^1$ représente un atome d'hydrogène.

20. Procédé selon l'une quelconque des revendications 1 à 19, dans lequel, dans la formule générale II, la valeur moyenne de m+n, est de 1 à 7.